(19) Europäisches Patentamt
European Patent Office
Office européen des brevets

(11) Veröffentlichungsnummer: 0 026 873
B1

# (12) EUROPÄISCHE PATENTSCHRIFT

(45) Veröffentlichungstag der Patentschrift: 06.10.82

(51) Int. Cl.³: C 07 D 261/18, A 01 N 43/80

(21) Anmeldenummer: 80105706.8

(22) Anmeldetag: 23.09.80

(54) **Isoxazolylcarbonsäureanilide, ihre Herstellung, diese enthaltende fungizide Mittel und Verfahren zur Bekämpfung von Pilzen.**

(30) Priorität: 04.10.79 DE 2940189

(43) Veröffentlichungstag der Anmeldung: 15.04.81 Patentblatt 81/15

(45) Bekanntmachung des Hinweises auf die Patenterteilung: 06.10.82 Patentblatt 82/40

(84) Benannte Vertragsstaaten: AT BE CH DE FR GB IT LI LU NL SE

(56) Entgegenhaltungen: DE-A-2 513 732

(73) Patentinhaber: BASF Aktiengesellschaft, Carl-Bosch-Strasse 38, D-6700 Ludwigshafen (DE)

(72) Erfinder: Zeeh, Bernd, Dr., Thorwaldsenstrasse 5, D-6700 Ludwigshafen (DE)
Erfinder: Theobald, Hans, Dr., Parkstrasse 2, D-6703 Limburgerhof (DE)
Erfinder: Ammermann, Eberhard, Dr., Sachsenstrasse 3, D-6700 Ludwigshafen (DE)
Erfinder: Pommer, Ernst-Heinrich, Dr., Berliner Platz 7, D-6703 Limburgerhof (DE)

EP 0 026 873 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Isoxazolylcarbonsäureanilide, ihre Herstellung, diese enthaltende fungizide Mittel und Verfahren zur Bekämpfung von Pilzen

Die vorliegende Erfindung betrifft neue Isoxazolylcarbonsäureanilide, Verfahren zu ihrer Herstellung, Fungizide, die diese Verbindungen als Wirkstoffe enthalten sowie Verfahren zur Bekämpfung von Pilzen.

Heterocyclische Carbonsäureanilide, die fungizid wirksam sind, sind aus der DE-A-2 513 732 und der DE-A-2 513 788 bekannt. Heterocyclische Reste in diesen Verbindungen sind Pyridyl, Pyrimidinyl, Dihydro-pyranyl, Dihydro-1,4-oxathiinyl, Thienyl und Furyl. Die Wirkung dieser Verbindungen gegen echte Mehltaupilze ist unzureichend.

Es wurde nun gefunden, daß 1,2-Isoxazolylcarbonsäureanilide der Formel I

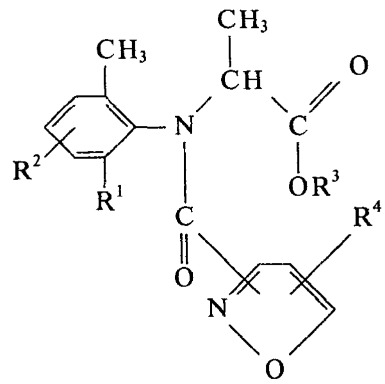

(I)

in der

$R^1$ Methyl, Ethyl oder Chlor,
$R^2$ Wasserstoff, Methyl, Ethyl, Fluor, Chlor oder Brom,
$R^3$ Methyl oder Ethyl und
$R^4$ Wasserstoff, Methyl oder Ethyl

bedeuten, fungizid ausgezeichnet wirksam sind und bekannten heterocyclischen Carbonsäureaniliden in ihrer Wirkung, insbesondere gegen Mehltaupilze, überlegen sind.

Die Isoxazolylcarbonsäureanilide der Formel I besitzen ein Asymmetriezentrum im Propionsäureesterrest. Die optisch reinen Enantiomeren können nach üblichen Methoden erhalten werden. Fungizid wirksam sind sowohl die bei der Synthese üblicherweise anfallenden Gemische als auch die reinen Enantiomeren, die von der Erfindung umfaßt werden.

Die Isoxazolylcarbonsäureanilide der Formel I können durch Umsetzung eines Anilinderivates der Formel II

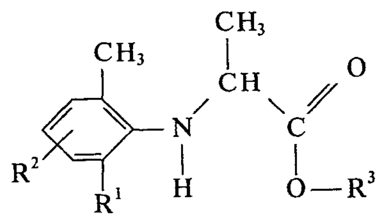

(II)

in der $R^1$, $R^2$ und $R^3$ die oben genannten Bedeutungen haben, mit einem Isoxazolylcarbonsäurederivat der Formel III

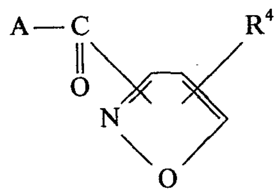

(III)

in der $R^4$ für Wasserstoff, Methyl oder Ethyl und A für eine nucleophil verdrängbare Abgangsgruppe steht, gegebenenfalls in Gegenwart eines Lösungs- oder Verdünnungsmittels, gegebenenfalls in Anwesenheit anorganischer oder organischer Basen und gegebenenfalls in Anwesenheit eines

Reaktionsbeschleunigers bei einer Temperatur im Bereich zwischen 0 und 120° C erhalten werden.

In Formel III bedeutet A beispielsweise Halogen, wie Chlor oder Brom, Alkoxycarbonyloxyreste, wie Methoxycarbonyloxy und Ethoxycarbonyloxy, oder den Benzyloxycarbonyloxyrest oder einen Azolylrest, wie den Imidazolyl- oder den Triazolylrest.

Die Umsetzung kann in Gegenwart eines Lösungs- oder Verdünnungsmittels durchgeführt werden. Zu den bevorzugten Lösungs- bzw. Verdünnungsmitteln gehören Halogenkohlenwasserstoffe, beispielsweise Methylenchlorid, Chloroform, 1,2-Dichloräthan, Chlorbenzol, aliphatische oder aromatische Kohlenwasserstoffe, wie Cyclohexan, Petroläther, Benzol, Toluol oder Xylole; Ester, wie Essigsäureäthylester; Nitrile, wie Acetonitril; Sulfoxide, wie Dimethylsulfoxid; Ketone, wie Aceton oder Methyläthylketon; Äther, wie Diäthyläther, Tetrahydrofuran oder Dioxan oder Gemische dieser Lösungsmittel.

Zweckmäßig verwendet man das Lösungs- bzw. Verdünnungsmittel in einer Menge von 100 bis 2000 Gew.-%, vorzugsweise 100 bis 1000 Gew.-%, bezogen auf die Ausgangsstoffe der Formel II bzw. III.

Geeignete anorganische oder organische Basen, die gegebenenfalls als säurebindende Mittel dem Reaktionsgemisch zugesetzt werden können, sind beispielsweise Alkalicarbonate, wie Kalium- oder Natriumcarbonat; Alkalihydride, wie Natriumhydrid oder tertiäre Amine, wie Trimethylamin, Triäthylamin, N,N-Dimethylanilin, N,N-Dimethylcyclohexylamin, N-Methylpiperidin oder Pyridin; Azole, wie 1,2,4-Triazol oder Imidazol. Es können aber auch andere übliche Basen verwendet werden.

Als Reaktionsbeschleuniger kommen vorzugsweise Metallhalogenide, wie Natriumbromid oder Kaliumjodid, Azole, wie Imidazol oder 1,2,4-Triazol oder Pyridine, wie 4-Dimethylaminopyridin oder Mischungen dieser Substanzen in Betracht. Zweckmäßigerweise setzt man auf 1 Mol Anilinderivat der Formel II 0,9 bis 1,3 Mol Säurederivat der Formel III sowie gegebenenfalls 0,5 bis 2 Mol Base und gegebenenfalls 0,01 Mol bis 0,1 Mol Reaktionsbeschleuniger ein.

Die Umsetzung wird im allgemeinen bei einer Temperatur im Bereich zwischen 0 und 120° C in einem Zeitraum von 1 bis 60 Stunden durchgeführt, drucklos oder unter Druck, kontinuierlich oder diskontinuierlich.

In einer bevorzugten Form des erfindungsgemäßen Verfahrens vermischt man den Ausgangsstoff der Formel II gegebenenfalls mit einer Base und gegebenenfalls mit einem Verdünnungsmittel, gibt dann das Säurederivat der Formel III und gegebenenfalls den Reaktionsbeschleuniger zu und hält das Reaktionsgemisch für 0,5 bis 12, vorzugsweise 1 bis 6 Stunden bei der Reaktionstemperatur, die zwischen 0 und 120° C liegen kann.

Zur Isolierung der neuen Verbindungen wird, falls vorhanden, das Verdünnungsmittel entfernt, der Rückstand wird dann in einem geeigneten Lösungsmittel gelöst und mit verdünnter Säure, dann mit wäßriger verdünnter Lauge sowie mit Wasser gewaschen, um die überschüssige Base und die Ausgangsstoffe II und III zu entfernen.

Die nach dem Abdestillieren des Lösungsmittels verbleibenden Produkte bedürften im allgemeinen keiner weiteren Reinigung, können aber, falls erforderlich, nach bekannten Methoden, beispielsweise durch Umkristallisation, Extraktion oder Chromatographie, weiter gereinigt werden.

Die Anilinderivate der Formel II sowie Verfahren zu ihrer Herstellung sind aus der DE-A-2 802 211, J. Org. Chem. 30, 4101–4104 (1965) und Tetrahedron 1967, 487–493 bekannt.

Die als Ausgangsstoffe eingesetzten Isoxazolylcarbonsäurederivate der Formel III sind teilweise bekannt bzw. können nach bekannten Methoden aus den Isoxazolylcarbonsäuren der Formel IV

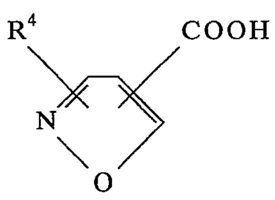

(IV)

in der $R^4$ für Wasserstoff, Methyl oder Ethyl steht, hergestellt werden.

Die Carbonsäuren der Formel IV sind teilweise bekannt (Gazz. chim. ital. 72, 458–474 (1942), ibid 73, 764–768 (1948)) oder können durch Oxidation der bekannten Carbinole (Gazz. chim. ital. 69, 536–539 (1939)) hergestellt werden.

Die erfindungsgemäßen Wirkstoffe weisen eine starke fungitoxische Wirkung auf. Sie schädigen Kulturpflanzen in den zur Bekämpfung von Pilzen und Bakterien notwendigen Konzentrationen nicht. Aus diesen Gründen sind sie für den Gebrauch als Pflanzenschutzmittel zur Bekämpfung von Pilzen geeignet.

Die neuen Wirkstoffe zeigen eine starke fungitoxische Wirksamkeit gegen phytopathogene Pilze. Sie sind beispielsweise geeignet zur Bekämpfung von Erysiphe graminis (echter Mehltau) in Getreide, Erysiphe cichoriacearum (echter Mehltau) an Kürbisgewächsen, Erysiphe polygoni an Bohnen, Podosphaera leucotridia und Phytophthora cactorum an Äpfeln.

Phytophthora infestans an Tomaten und Kartoffeln, Phytophthora parasitica an Erdbeeren, Pseudoperonospora cubensis an Gurken, Pseudoperonospora humuli an Hopfen, Peronospora

destructor an Zwiebeln, Peronospora tabacina an Tabak, Peronospora sparsa an Rosen, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Uncinula necator an Reben und Sphaerotheca pamosa an Rosen.

Die Aufwandmengen liegen je nach Art des gewünschten Effektes zwischen 0,1 und 5 kg Wirkstoff je ha. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen. Darüber hinaus sind viele der neuen Verbindungen systemisch wirksam, so daß über die Wurzelbehandlung auch ein Schutz oberirdischer Pflanzen möglich ist.

Ferner lassen sich mit den neuen Verbindungen auch Pilze, die Keimlings- und Auflaufkrankheiten hervorrufen, beispielsweise Pythium- und Aphanomyces-Arten an Leguminosen und Baumwolle, bekämpfen. Die Aufwandmengen betragen je 100 kg Saatgut 10 bis 200 g Wirkstoff; die Anwendung erfolgt in Form von Saatgutbeizmitteln.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemäßen Substanzen können in die üblichen Formulierungen wie Lösungen, Emulsionen, Suspensionen, Stäube, Pulver, Pasten und Granulate übergeführt werden. Die Anwendungsformen richten sich ganz nach den Verwendungszwecken; sie sollten in jedem Fall eine feine und gleichmäßige Verteilung der wirksamen Substanzen gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen — Fettalkohol — Ether, Alkylsulfonate und Arylsulfonate) und Dispergiermittel, wie Lignin, Sulfitablaugen und Methylcellulose in Betracht.

Die fungiziden Mittel enthalten 0,1 bis 95 Gew.-% Wirkstoff, vorzugsweise 0,5 bis 90 Gew.-%.

Die Mittel bzw. die daraus hergestellten gebrauchsfertigen Zubereitungen, wie Lösungen, Emulsionen, Suspensionen, Pulver, Stäube, Pasten oder Granulate, werden in bekannter Weise angewendet, beispielsweise durch Versprühen, Vernebeln, Verstäuben, Verstreuen, Beizen oder Gießen.

Die erfindungsgemäßen Mittel können in diesen Anwendungsformen auch zusammen mit anderen Wirkstoffen vorliegen, wie z. B. Herbiziden, Insektiziden, Wachstumsregulatoren und anderen Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Dithiocarbamate und deren Derivate, wie
- Ferridimethyldithiocarbamat,
- Zinkdimethyldithiocarbamat,
- Manganethylenbisdithiocarbamat,
- Mangan-Zink-ethylendiamin-bis-dithiocarbamat,
- Zinkethylenbisdithiocarbamat,
- Tetramethylthiuramdisulfide,
- Ammoniak-Komplex von Zink-(N,N-ethylen-bis-dithiocarbamat) und N,N'-Polyäthylen-bis-(thiocarbamoyl)-disulfid,
- Zink-(N,N'-propylen-bis-dithiocarbamat),
- Ammoniak-Komplex von Zink-(N,N'-propylen-bis-dithiocarbamat) und N,N'-Polypropylen-bis-(thiocarbamoyl)-disulfid;

Nitroderivate, wie
- Dinitro-(1-methylheptyl)-phenylcrotonat,
- 2-sec-Butyl-4,6-dinitrophenyl-3,3-dimethylacrylat,
- 2-sec-Butyl-4,6-dinitrophenyl-isopropylcarbonat;

heterocyclische Strukturen, wie
- N-Trichlormethylthio-tetrahydrophthalamid,
- N-Trichlormethylthio-phthalimid,

2-Heptadecyl-2-imidazolin-acetat,
2,4-Dichlor-6-(o-chloranilino)-s-triazin,
0,0-Diäthyl-phthalimidophosphonothionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-3-pyridinmethanol,
1,2-Bis-(3-ethoxycarbonyl-2-thioureido)-benzol,
1,2-Bis-(3-methoxycarbonyl-2-thioureido)-benzol

und verschiedene Fungizide, wie

Dodecylguanidinacetat,
3-(3-(3,5-Dimethyl-2-oxycyclohexyl)-2-hydroxyäthyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze.

5-Nitro-isophthalsäure-di-isopropylester,

1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(n-Propyl)-N-(2,4,6-trichlorphenoxyethyl)-N'-imidazolyl-harnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin.

## Experimenteller Teil

### 1) Herstellung von Ausgangsisoxazolylcarbonsäuren

Am Beispiel der 3-Ethylisoxazolylcarbonsäure-(5) wird die Herstellung der Isoxazolylcarbonsäuren der Formel IV aus den entsprechenden Carbinolen durch Oxidation erläutert:

63,5 g 3-Ethyl-5-hydroxymethylisoxazol in 250 ml Diethylether werden bei 15 bis 20°C mit einer Mischung aus 750 ml Wasser, 164 g $Na_2Cr_2O_7 \cdot 2\ H_2O$ und 124 ml Schwefelsäure tropfenweise versetzt. Nach 12stündigem Rühren bei 20 bis 250°C wird die organische Phase abgetrennt und über Natriumsulfat getrocknet. Nach Entfernung des Lösungsmittels erhält man 59 g 3-Ethylisoxazolylcarbonsäure-(5) vom Schmelzpunkt 194°C.

$C_6H_7NO_3$ (141)

| | | | |
|---|---|---|---|
| Ber.: | C 51,1 | H 5,0 | N 9,9 |
| Gef.: | C 51,9 | H 5,2 | N 10,7 |

Die folgende Tabelle gibt eine Übersicht über die Schmelzpunkte einiger Carbonsäuren der Formel IV:

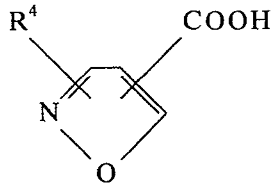

(IV)

Tabelle

| $R^4$ | Stellung der COOH-Gruppe am Isoxazolring | Fp. [°C] |
|---|---|---|
| H | 3 | 149 |
| H | 4 | 123 |
| H | 5 | 144 |
| 5-$CH_3$ | 3 | 176 |
| 3-$CH_3$ | 5 | 211 |

Das folgende Beispiel erläutert die Herstellung der Isoxazolylcarbonsäureanilide der Formel I. Gewichtsteile verhalten sich zu Volumenteilen wie kg zu l.

2) Synthesebeispiel für 3-Methyl-isoxazolyl-5-carbonsäure-N-(1'-methoxycarbonylethyl)-(2'',6''-dimethyl-anilid)

Zu einer Lösung von 20,7 Gew.-Teilen N-(1-Methoxycarbonylethyl)-2',6'-dimethylanilin in 100 Volumenteilen Toluol werden 15,2 Volumenteile Triethylamin und anschließend eine Lösung von 16 Gew.-Teilen 3-Methyl-isoxazolylcarbonsäure-5-chlorid in 30 Volumenteilen Toluol getropft. Die Temperatur der Reaktionsmischung steigt dabei auf 64°C an. Nach 2 Stunden Rühren bei Raumtemperatur werden 50 Volumenteile Wasser zugegeben. Die organische Phase wird abgetrennt, dreimal mit je 50 Volumenteilen Wasser gewaschen, anschließend getrocknet und eingeengt. Das verbleibende Öl wird unter vermindertem Druck destilliert.

Bei 0,005 mbar und 170 bis 174°C werden 24,1 Gew.-Teile 3-Methyl-isoxazolyl-5-carbonsäure-N-(1'-methoxycarbonylethyl)-(2'',6''-dimethyl-anilid) erhalten (Wirkstoff Nr. 1).

$C_{17}H_{20}O_4N_2$ (316,3)

| | | | |
|---|---|---|---|
| Ber.: | C 64,5 | H 6,4 | N 8,9 |
| Gef.: | C 64,6 | H 6,3 | N 8,9 |

Analog lassen sich beispielsweise folgende Verbindungen der Formel I herstellen:

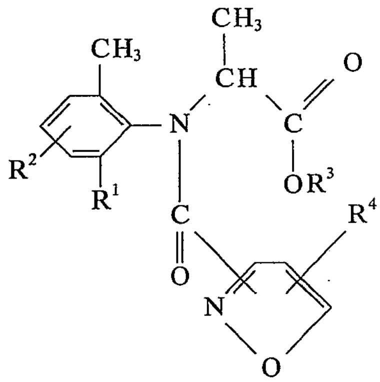

(I)

| Nr. | $R^1$ | $R^2$ | $R^3$ | $R^4$ | Position der CO-Gruppe am Isoxazolring | Phys. Daten |
|---|---|---|---|---|---|---|
| 2 | $CH_3$ | H | $CH_3$ | 3-$C_2H_5$ | 5 | Kp. 170–174°C/ 0,01 mbar |
| 3 | $CH_3$ | H | $C_2H_5$ | 3-$C_2H_5$ | 5 | Öl |
| 4 | $CH_3$ | H | $CH_3$ | H | 3 | Fp. 75–77° C |
| 5 | Cl | H | $CH_3$ | H | 3 | Fp. 85°C |
| 6 | $CH_3$ | 3-$CH_3$ | $CH_3$ | H | 3 | Öl |
| 7 | $CH_3$ | 4-Br | $CH_3$ | H | 3 | Fp. 85° C |
| 8 | $CH_3$ | H | $CH_3$ | H | 4 | |
| 9 | Cl | H | $CH_3$ | H | 4 | |
| 10 | $CH_3$ | 3-$CH_3$ | $CH_3$ | H | 4 | |
| 11 | $CH_3$ | 3-$CH_3$ | $CH_3$ | H | 5 | $n_D^{20}$ 1,5310 |
| 12 | Cl | H | $CH_3$ | H | 5 | Fp. 108–110° C |
| 13 | $CH_3$ | H | $CH_3$ | H | 5 | Fp. 88–90° C |
| 14 | $C_2H_5$ | H | $CH_3$ | 3-$CH_3$ | 5 | $n_D^{20}$ 1,5250 |
| 15 | $C_2H_5$ | H | $CH_3$ | H | 3 | $n_D^{20}$ 1,526 |

3) Beispiele für Zubereitungen

I. Man vermischt 90 Gew.-Teile des Wirkstoffs Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung des Wirkstoffs Nr. 1 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.

III. 20 Gew.-Teile des Wirkstoffs Nr. 4 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in

100 000 Gew.-Teilen Wasser erhält man eine wäßrige Dispersion, die 0,02 Gew.-% des Wirkstoffs enthält.

IV. 20 Gew.-Teile des Wirkstoffs Nr. 2 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

V. 20 Gew.-Teile des Wirkstoffs Nr. 5 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.-% des Wirkstoffs enthält.

VI. 3 Gew.-Teile des Wirkstoffs Nr. 3 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile des Wirkstoffs Nr. 4 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufarbeitung des Wirkstoffs mit guter Haftfähigkeit.

VIII. 40 Gew.-Teile des Wirkstoffs Nr. 2 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gew.-Teilen Wasser erhält man eine wäßrige Dispersion, die 0,04 Gew.-% Wirkstoff enthält.

IX. 20 Teile des Wirkstoffs Nr. 4 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

## 4) Fungizide Wirksamkeit

Die folgenden Beispiele belegen die biologische Wirkung der neuen Verbindungen der Formel I. Vergleichsmittel sind die fungiziden Wirkstoffe N-(1'-Methoxycarbonyl-ethyl)-N-(furan-(2'')-carbonyl)-2,6-dimethylanilin (A; DE-A-2 513 788) und N-(1'-Methoxycarbonylethyl)-N-(pyridin-3''-carbonyl)-2-methyl-6-chloranilin (B; DE-A-2 513 732).

### Beispiel A

### Wirksamkeit gegen Weizenmehltau

Blätter von in Töpfen gewachsenen Weizenkeimlingen der Sorte »Jubilar« werden mit wäßrigen Emulsionen aus 80% (Gew.-%) Wirkstoff und 20% Emulgiermittel besprüht und nach dem Antrocknen des Spritzbelags mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltaupilzentwicklung ermittelt.

| Wirkstoff | Befall der Blätter nach Spritzungen mit x%iger Wirkstoffbrühe | | | |
|---|---|---|---|---|
| | x = 0,05 | 0,025 | 0,012 | 0,006 |
| 1 | 0 | 2 | 2–3 | 4 |
| 2 | 0 | 1 | 2 | 2–3 |
| 3 | 0 | 0 | 2 | 2–3 |
| 4 | 0 | 0 | 1 | 2 |
| Vergleichsmittel A | 5 | 5 | – | – |
| Vergleichsmittel B | 4 | 5 | 5 | 5 |
| Kontrolle (unbehandelt) | 5 | | | |

0 = kein Pilzbefall, abgestuft bis 5 Totalbefall.

Beispiel B

Wirksamkeit gegen Phytophthora an Tomaten

Blätter von Tomatenpflanzen der Sorte »Professor Rudloff« werden mit wäßrigen Suspensionen, die 80% (Gew.-%) des zu prüfenden Wirkstoffes und 20% Natriumligninsulfonat in der Trockensubstanz enthalten, besprüht. Es werden 0,1, 0,05, 0,025 und 0,012%ige Spritzbrühen (berechnet auf die Trockensubstanz) verwendet. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

| Wirkstoff | Befall der Blätter nach Spritzungen mit x%iger Wirkstoffbrühe | | | |
|---|---|---|---|---|
| | x = 0,1 | 0,05 | 0,025 | 0,012 |
| 1 | 0 | 1 | 2 | 2 |
| 4 | 0 | 0 | 0 | 2 |
| 5 | 0 | 0 | 1 | 1 |
| 6 | 0 | 0 | 0 | 0 |
| 13 | 0 | 0 | 0 | 1 |
| Vergleichsmittel A | 1 | 2 | 3–4 | 4 |
| Vergleichsmittel B | 2 | 3 | 4 | 4 |
| Kontrolle (unbehandelt) | 5 | | | |

O = kein Pilzbefall, abgestuft bis 5 Totalbefall.

Beispiel C

Wirksamkeit gegen Rebenperonospora

Blätter von Topfreben der Sorte »Müller-Thurgau« werden mit wäßrigen Emulsionen, die 80% (Gew.-%) des zu prüfenden Wirkstoffs und 20% Emulgiermittel enthalten, besprüht. Es werden 0,020- und 0,012%ige Spritzbrühen (bezogen auf die Trockensubstanz) verwendet. Um die Wirkungsdauer der Wirkstoffe beurteilen zu können, werden die Pflanzen nach dem Antrocknen des Spritzbelags 10 Tage im Gewächshaus aufgestellt. Erst dann werden die Blätter mit einer Zoosporenaufschwemmung von Plasmopara viticola (Rebenperonospora) infiziert. Danach kommen die Reben zunächst für 16 Stunden in eine wasserdampfgesättigte Kammer bei 24°C und anschließend 8 Tage in ein Gewächshaus mit Temperaturen zwischen 20 und 30°C. Nach dieser Zeit werden die Pflanzen zur Beschleunigung des Sporangienträgerausbruchs abermals während 16 Stunden in der feuchten Kammer aufgestellt. Dann erfolgt die Beurteilung des Ausmaßes des Pilzausbruchs auf den Blattunterseiten.

| Wirkstoff | Befall der Blätter nach Spritzung mit x%iger Wirkstoffbrühe | |
|---|---|---|
| | x= 0,025 | 0,012 |
| 1 | 0 | 0 |
| 2 | 0 | 0 |
| Kontrolle (unbehandelt) | 5 | |

0 = kein Pilzbefall, abgestuft bis 5 Totalbefall.

**Patentansprüche**

1. Isoxazolylcarbonsäureanilide der Formel I

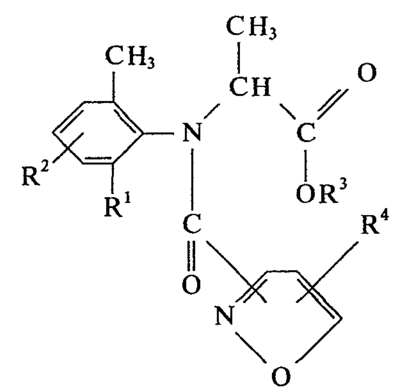

(I)

in der

$R^1$ Methyl, Ethyl oder Chlor,
$R^2$ Wasserstoff, Methyl, Ethyl, Fluor, Chlor oder Brom,
$R^3$ Methyl oder Ethyl und
$R^4$ Wasserstoff, Methyl oder Ethyl bedeuten.

2. Isoxazolyl-3-carbonsäure-N-(1′-methoxy-carbonylethyl)-(2″,6″-dimethyl)-anilid.

3. 3-Methyl-isoxazolyl-5-carbonsäure-N-(1′-methoxycarbonylethyl)-(2″,6″-dimethyl)-anilid.

4. Verfahren zur Herstellung von Isoxazolylcarbonsäureaniliden der Formel I gemäß Anspruch 1, dadurch gekennzeichnet, daß man ein Anilinderivat der Formel II

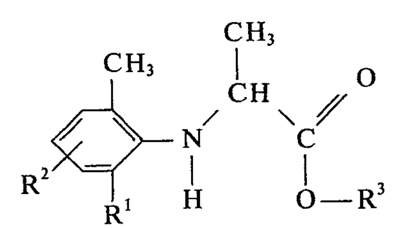

(II)

in der $R^1$, $R^2$ und $R^3$ die in Anspruch 1 genannten Bedeutungen haben, mit einem Isoxazolylcar-

bonsäurederivat der Formel III

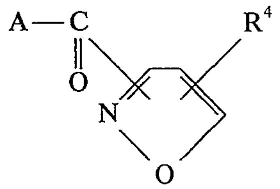

(III)

in der

$R^4$ die im Anspruch 1 genannten Bedeutungen hat und
A für eine nucleophil verdrängbare Abgangsgruppe steht, gegebenenfalls in Gegenwart eines Lösungs- oder Verdünnungsmittels, gegebenenfalls in Anwesenheit anorganischer oder organischer Basen und gegebenenfalls in Anwesenheit eines Reaktionsbeschleunigers bei einer Temperatur im Bereich zwischen 0 und 120° C umsetzt.

5. Fungizide Mittel, enthaltend mindestens ein Isoxazolylcarbonsäureanilid der Formel I gemäß Anspruch 1.

6. Fungizide Mittel, enthaltend mindestens ein Isoxazolylcarbonsäureanilid der Formel I gemäß Anspruch 1 und einen festen oder flüssigen Trägerstoff.

7. Verfahren zur Bekämpfung von Pilzen, dadurch gekennzeichnet, daß man mindestens ein Isoxazolylcarbonsäureanilid der Formel I gemäß Anspruch 1 auf diese bzw. auf durch Pilzbefall bedrohte Flächen, Pflanzen oder Saatgüter einwirken läßt.

**Claims**

1. An isoxazolylcarboxanilide of the formula I

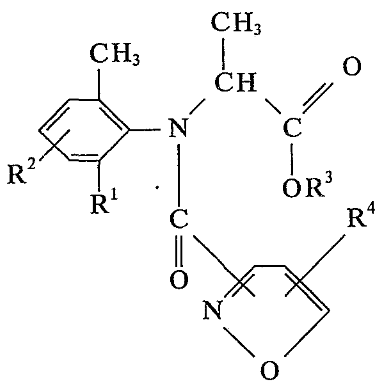

(I)

where $R^1$ is methyl, ethyl or chlorine, $R^2$ is hydrogen, methyl, ethyl, fluorine, chlorine or bromine, $R^3$ is methyl or ethyl, and $R^4$ is hydrogen, methyl or ethyl.

2. Isoxazolyl-3-carboxylic acid-N-(1′-methoxycarbonylethyl)-(2″,6″-dimethyl)-anilide.

3. 3-Methylisoxazolyl-5-carboxylic acid-N-(1′-methoxycarbonylethyl)-(2″,6″-dimethyl)-anilide.

4. A process for the production of an isoxazolylcarboxanilide of the formula I as claimed in claim 1, characterized in that an aniline derivative of the formula II

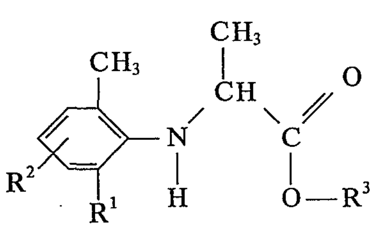

(II)

where $R^1$, $R^2$ and $R^3$ have the meanings given in claim 1, is reacted with an isoxazolylcarboxylic acid derivative of the formula III

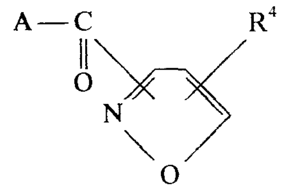

(III)

where $R^4$ has the meaning given in claim 1 and A denotes a nucleophilically displaceable leaving group, in the presence or absence of a solvent or diluent, in the presence or absence of inorganic or organic bases, and in the presence or absence of a reaction accelerator, at a temperature of from 0° to 120° C.

5. A fungicidal agent containing at least one isoxazolylcarboxanilide of the formula I as claimed in claim 1.

6. A fungicidal agent containing at least one isoxazolylcarboxanilide of the formula I as claimed in claim 1 and a solid or liquid carrier.

7. A process for combating fungi, characterized in that at least one isoxazolylcarboxanilide of the formula I as claimed in claim 1 is allowed to act on the fungi, or on areas, plants or seed threatened by fungus attack.

**Revendications**

1. Anilides d'acide isoxazolylcarboxylique de formule I

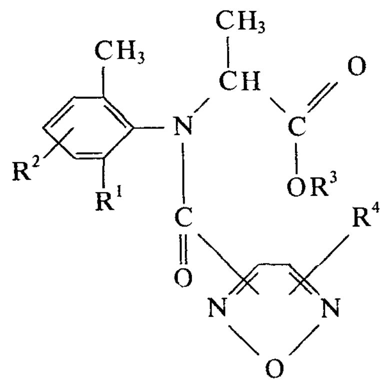

(I)

dans laquelle

$R^1$ représente méthyle, éthyle ou chlore,
$R^2$ hydrogène, méthyle, éthyle, fluor, chlore ou brome,
$R^3$ méthyle ou éthyle et
$R^4$ hydrogène, méthyle ou éthyle.

2. N-(1'-méthoxy-carbonyléthyl)-(2'',6''-diméthyl)-anilide d'acide isoxazolyl-3-carboxylique.

3. N-(1'-méthoxycarbonyléthyl)-(2'',6''-diméthyl)-anilide d'acide 3-méthyl-isoxazolyl-5-carboxylique.

4. Procédé de préparation d'anilides d'acide isoxazolylcarboxylique de formule I, selon la revendication 1, caractérisé par le fait que l'on fait réagir à une température comprise entre 0 et 120° C un dérivé d'aniline de formule II

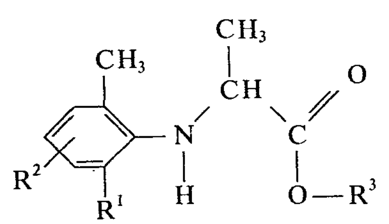

(II)

dans laquelle $R^1$, $R^2$ et $R^3$ ont la signification indiquée à la revendication 1 avec un dérivé d'acide isoxazolylcarboxylique de formule III

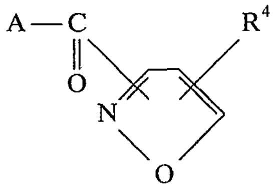

(III)

dans laquelle

$R^4$ a la signification indiquée dans la revendication 1 et
A représente un groupe de départ nucléophile, déplaçable éventuellement en présence d'un solvant ou diluant, éventuellement en présence de bases inorganiques ou organiques et éventuellement en présence d'un accélérateur de réaction.

5. Agent fongicide contenant au moins un anilide d'acide d'isoxazolylcarboxylique de formule I selon la revendication 1.

6. Agent fongicide, contenant au moins un anilide d'acide isoxazolylcarboxylique de formule I selon la revendication 1 et un support solide ou liquide.

7. Procédé de lutte contre les champignons, caractérisé par le fait qu'on fait agir au moins un anilide d'acide isoxazolylcarboxylique de formule I selon la revendication 1 sur ceux-ci ou sur des surfaces, plantes ou graines menacées par une maladie cryptogamique.